(19) Europäisches Patentamt
European Patent Office
Office européen des brevets

(11) Publication number: 0 330 824 A1

(12) EUROPEAN PATENT APPLICATION

(21) Application number: 89100964.9

(22) Date of filing: 20.01.89

(51) Int. Cl.4: A61K 31/435 , A61K 31/47

(30) Priority: 01.03.88 EP 88400482

(43) Date of publication of application:
06.09.89 Bulletin 89/36

(84) Designated Contracting States:
AT BE CH DE ES FR GB GR IT LI LU NL SE

(71) Applicant: MERRELL DOW PHARMACEUTICALS INC.
2110 East Galbraith Road
Cincinnati Ohio 45215-6300(US)

(72) Inventor: Gittos, Maurice Ward
16 Rue André Malraux
F-67115 Plobsheim(FR)

(74) Representative: Macchetta, Francesco et al
Gruppo Lepetit S.p.A. Patent and Trademark
Department 34, Via Roberto Lepetit
I-21040 Gerenzano (Varese)(IT)

(54) **Use of quinolizine and quinolizinone derivatives in the manufacture of medicaments for the treatment of cardiac arrhythmias.**

(57) The present invention is directed to the use of hetrocyclic esters of hexahydro-8-hydroxy-2,6-methano-2H-quinolizin-3(4H)-one and hexahydro-8-hydroxy-2,6-methano-2H-quinolizines as cardiac antiarrhythmic agents.



Xerox Copy Centre

## Use of Quinolizine and Quinolizinone Derivatives in the Manufacture of Medicaments for the Treatment of Cardiac Arrhythmias

The present invention is directed to the use of a class of compounds in the manufacture of a medicament for the treatment of cardiac arrhythmias.

In accordance with the present invention it has been discovered that cardiac arrhythmias can be treated, in a patient in need thereof, by the administration of an antiarrhythmic quantity of a compound of the formula:

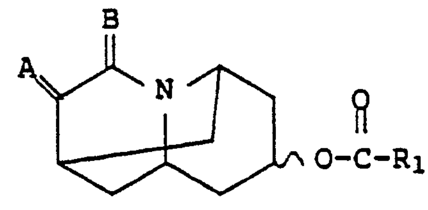

Formula I

wherein A is $H_2$, 0, (H)(OH), $(OH)_2$ or N-OH; B is $H_2$, $(H)(CH_3)$, $(H)(CH_2NR_3R_4)$ or $CH_2$ wherein $R_3$ and $R_4$ are $C_{2-4}$ alkyl or are combined to give tetramethylene, pentamethylene or $-CH_2CH_2-0-CH_2CH_2$ ; $R_1$ is

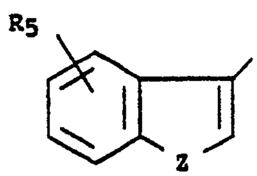 , 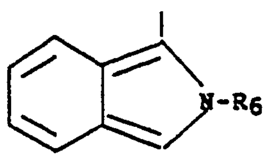 , 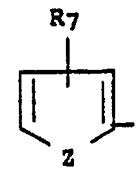

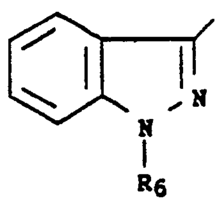 or 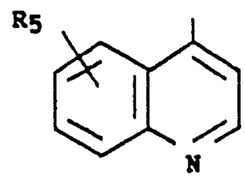

wherein Z is $NR_6$, O or S; $R_6$ is hydrogen, $C_{1-4}$ alkyl or phenyl ($C_{1-2}$ alkyl); $R_5$ is hydrogen, halogen, $C_{1-4}$ alkyl, $C_{1-4}$ alkoxy, hydroxy, cyano or $-CONH_2$; $R_7$ is hydrogen, halogen, $C_{1-4}$ alkyl or phenyl; the wavy line indicates that the configuration of the oxygen substituent on the ring can be endo or exo; and the pharmaceutically acceptable acid addition and quaternary ammonium salts of the aforesaid compounds.

The compounds of the above formula (formula I) wherein

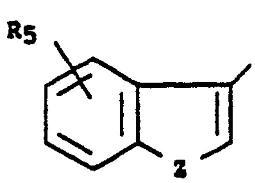 , 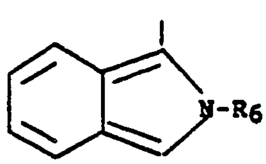 , 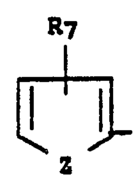

are defined as above, are described in EP-A-166730. They are taught to be useful in the treatment of pain, nausea and vomiting.

Those compounds of formula I wherein $R_1$ represents

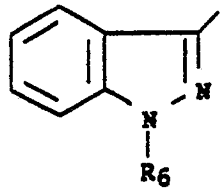

or

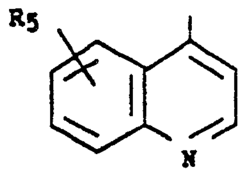

wherein $R_5$ and $R_6$ are as defined above are described in a recently filed copending application (our ref.: MDE 6097 filed on 11.01.89).

Examples of the $C_{1-4}$ alkyl groups referred to above are methyl, ethyl, propyl, isopropyl, and butyl. Examples of the $C_{1-4}$ alkoxy groups are methoxy, ethoxy, propoxy and butoxy. The halogens referred to above can be fluorine, chlorine or bromine. When the wavy line in the general structural formula is changed to a solid line, this indicates that the configuration of the compounds is endo. Such endo-compounds can also be referred to as trans. Similarly, exo-compounds can also be referred to as cis. Any hydrates of the present compounds are considered as equivalent to the compounds themselves and this would include compounds in which the carbonyl (i.e., A is O) exists as $(OH)_2$.

A preferred group of compounds are those wherein the ester is attached to the polycyclic ring in the endo-configuration. A further preferred group are those having the endo-configuration wherein A is O and $(OH)_2$. In a still further preferred group, B is additionally $H_2$.

The pharmaceutically acceptable acid addition salts referred to above can be non-toxic salts with suitable acids such as those with inorganic acids, for example hydrochloric, hydrobromic, nitric, sulfuric or phosphoric acids; or with organic acids such as organic carboxylic acids, for example, acetic, propionic, glycolic, maleic, hydroxymaleic, malic, tartaric, citric, salicyclic, 2-acetyloxybenzoic, nicotinic or isonicotinic; or organic sulfonic acids, for example methanesulfonic, ethanesulfonic, 2-hydroxyethanesulfonic, 4-toluenesulfonic or 2-naphthalensulfonic. Quaternary ammonium salts are formed with alkyl halides such as methyl chloride, methyl bromide or ethyl bromide; or with sulfate esters such as methyl 4-toluenesulfonate or methyl 2-naphthalenesulfonate.

Some specific examples of compounds encompassed for use in the present invention are the following:

endo-8-(3-Indolylcarbonyloxy)octahydro-2,6-methano-2H-quinolizine
endo-8-(5-Cyano-3-indolylcarbonyloxy)hexahydro-2,6-methano-2H-quinolizin-3(4H)-one
endo-8-(3-Indolylcarbonyloxy)hexahydro-4-(diethylaminomethyl)-2,6-methano-2H-quinolizin-3(4H)-one
endo-8-(3-Indolylcarbonyloxy)-3-hydroxyimino-2,6-methanooctahydro-2H-quinolizine
endo-8-(2-Methyl-1-isoindolycarbonyloxy)hexahydro-2,6-methano-2H-quinolizin-3(4H)-one
endo-8-(2-Pyrrolidinylcarbonyloxy)hexahydro-2,6-methano-2H-quinolizin-3(4H)-one
endo-8-(3-Indolylcarbonyloxy)-2,6-methanooctahydro-2H-quinolizin-3-ol
endo-Hexahydro-8-(1-methyl-3-indazolylcarbonyloxy)-2,6-methano-2H-quinolizin-3(4H)-one
endo-Hexahydro-8-(3-indazolylcarbonyloxy)-2,6-methano-2H-quinolizin-3(4H)-one.

The compounds of the present invention can be prepared by reacting an alcohol or reactive derivatives thereof, said alcohol having the formula:

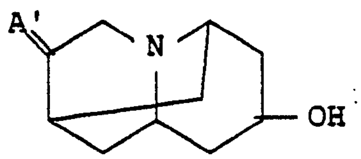

wherein $A'$ is O or $H_2$, with a reactive equivalent of an acid of the formula:

$R_1COOH$

wherein $R_1$ is defined as above. By a reactive equivalent of the acid is meant the corresponding acid chloride or bromide or the corresponding glyoxylyl chloride or bromide or the carboxylic acid imidazole obtained by the reaction of the appropriate acid halide with N,N-carbonyldiimidazole; or any similar acid derivative which would yield the simple carboxylic acid ester on reaction with an alcohol or with a reactive derivative of an alcohol. More specifically, where the -OH in the alcohol is equatorial (exo), then it can be reacted with the appropriate carboxylic acid imidazole obtained by the reaction of the acid halide with N,N-carbonyldiimidazole. Alternatively, the acid can be converted to the acid chloride by standard procedures (e.g., thionyl chloride) and then reacted with the alcohol or an alkali metal salt of the alcohol such as the

lithium salt obtained by the reaction of lithium hydride with the alcohol in tetrahydrofuran.

When the -OH group in the starting alcohol is axial (endo), it can also be converted to the corresponding ester by reaction with the appropriate acid chloride or bromide with the reaction being carried out in the presence of an equivalent of a suitable tertiary base such as 4-dimethylaminopyridine in a high boiling inert solvent such as xylene. In this case, however, long heating (24-84 hours) at a temperature at or above 140° C is necessary so that the procedure would not be suitable for use with acid halides that are not stable under the indicated conditions. Thus, it was necessary to use an alternative for the preparation of such compounds. In this procedure, an appropriate acid chloride or bromide or a glyoxylyl chloride or bromide, in a nitroparaffin solvent, is reacted with a solution of a super acid salt of the alcohol and an equivalent amount of a heavy metal salt of the same super acid. The glyoxylyl chloride can be used in the process as indicated because it decarbonylates readily under the conditions used. The reaction itself can be carried out over a period of 1-24 hours at temperatures ranging from -80° C to ambient temperatures (about 23° C). Examples of suitable super acids with M = H are $MBF_4$, $MAsF_6$, $MSbF_6$, $MPF_6$, $MTaF_6$ or $MNbF_6$ with examples of suitable heavy metals (M) being silver and thallium. Examples of nitroparaffin solvents are nitromethane, nitroethane, 1-nitropropane, and 2-nitropropane.

Actually, where the group RI contains a primary or secondary amino group, it is usually protected during the above reaction, with a benzyl group being commonly used to protect a secondary amine and a benzyloxycarbonyl group being used to protect a primary amine. In either case, the protecting group in the product is removed by conventional procedures, for example by hydrogenation with hydrogen and a palladium catalyst.

Various procedures can be used to convert those compounds wherein A is O and whose preparation is described below, to other different bridged derivatives of the present invention by standard methods. Thus, the ketone group in the polycyclic system can be reduced to the corresponding alcohol using an alkali metal (sodium or potassium) borohydride in a lower alkanol such as methanol or ethanol.

The ketone group can also be reduced completely to a methylene group by a two step procedure. In the first step, the ketone is reacted with ethylene dithiol or trimethylene dithiol in the presence of a strong acid such as hydrochloric acid or $BF_3$ to give the corresponding dithioketal. The reaction is carried out in a suitable polar solvent such as nitromethane or acetic acid. The dithioketal is then reduced with hydrazine in the presence of Raney nickel in a lower alkanol solvent such a 2-propanol at elevated temperatures (60-100° C). Actually this same procedure can be used to reduce the original starting alcohol, hexahydro-8-hydroxy-2,6-methano-2H-quinolizin-3(4H)-one, to 8-hydroxy-2,6-methanooctahydro-2H-quinolizine which can itself be reacted with acid derivatives as described earlier to give the corresponding esters.

Compounds containing other B-groups (i.e. aminomethyl, methylene or methyl groups) can be obtained from products in which A is O and B is $H_2$ by a Mannich reactions using formaldehyde and a secondary amine such as dimethylamine, diethylamine, piperidine or pyrrolidine. This reaction gives the corresponding aminomethyl compound and, when B is dimethylaminomethyl, the amino moiety is eliminated on heating at 90-110° C in an inert solvent such as toluene to give the corresponding methylene compound (B is $CH_2$). This exocyclic methylene compound can be isolated by standard methods and transformed into a methyl group by hydrogenation, for example, by using hydrogen and platinum oxide.

To obtain those compounds in which A is hydroxyimino(N-OH), the ketone referred to above can be reacted with hydroxylamine hydrochloride by standard procedures.

The alcohol used as a reactant in the above procedure can be obtained from known alkyl ($C_{1-4}$) 3-cyclopentene-1-carboxylates by a multi-step procedure. Specifically, the double bond in the indicated cyclopentene is oxidized to a 1,2-diol using N-methyl-morpholine N-oxide in the presence of osmium tetroxide catalyst. The diol is then cleaved to the corresponding dialdehyde using sodium metaperiodate. A Robinson-Schöpf cyclization of the dialdehyde with a lower alkyl glycine ester and acetone-dicarboxylic acid, preferably at $pH_4$, gives a pseudopelletierine derivative of the following type:

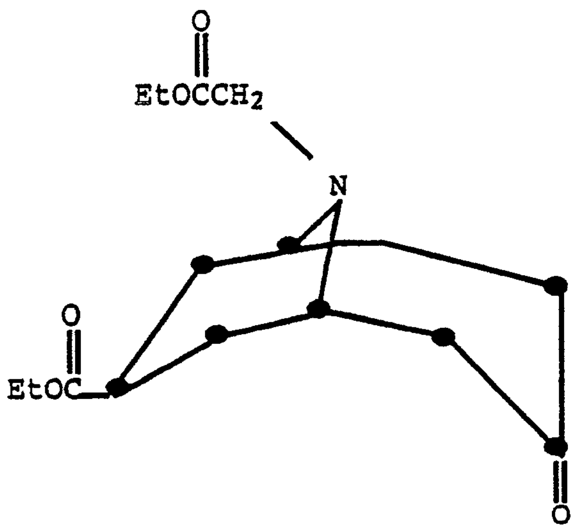

The ketone group is reduced to an alcohol using sodium borohydride and the product is reacted with dihydropyran to protect the -OH group as a tetrahydropyranyl ether. Dieckmann cyclization of the diester using a strong base (e.g. potassium t-butoxide) followed by aqueous acid hydrolysis and decarboxylation gives the desired alcohol. The resulting alcohols can exist in two conformations -axial and equatorial. The main product obtained by the above procedure is the axial alcohol and it can be separated from the equatorial isomer by crystallization of the camphorsulfonate or tetrafluoroborate salt.

The compounds represented by Formula I are 5-HT M-receptor antagonists which are useful in the treatment of cardiac arrhythmias and in preparing a medicament, therefor.

The activity of the compounds against 5-HT can be assessed by determining their $pA_2$ values in the isolated rabbit heart as described by J.R. Fozard et al., Eur. J. Pharmacol., 59, 195-210 (1979). In the method described, the molar concentration of antagonist which reduces the effects of twice the $ED_{50}$ of 5-HT to that of the $ED_{50}$ in the absence of antagonist is determined. The $pA_2$ value is the negative logarithm of said molar concentrations. In general terms, the higher the $pA_2$ value the more potent is the compound. When tested in this way, the present compounds show $pA_2$'s generally in the range of about 8 to 10.

The activity of these compounds against 5-HT can be assessed in vivo by measurement of the effect of the compound on the Von Bezold-Jarisch Reflex induced by 5-HT injected intravenously into the rat (see Paintal A.S., Physiol. Rev. 53, 159-227, 1973; J.R. Fozard, Naunyn-Schmiedeberg's Arch. Pharmacol., 326, 1984, 36-44). The transient cardiac slowing arises from an increased afferent vagus activity arising from stimulation by 5-HT of sensory afferent fibers in and around the heart. When tested against the Von Bezold-Jarisch Reflex induced by 5-HT, the compound endo-hexahydro-8-(3-indolylcarbonyloxy)-2,6-methano-2H-quinolizin-3(4H)-one hydrochloride suppressed the response dose-dependently at doses of 0.01-0.1 mg/kg given intravenously or 0.25-1 mg/kg given orally.

The present compounds appear to be highly selective in their action against the 5-HT M-receptor. Their potency against other 5-HT receptors and other spasmogens, in particular carbachol, phenylephrine, histamine and calcium, is known to be at least three orders lower that that against 5-HT M-receptors.

The compounds represented by Formula I are useful as cardiac antiarrhythmic agents. They can be administered to a patient suffering from an arrhythmic episode in order to terminate the arrhythmic episode and return the myocardium to a normal sinus rhythm or the compound can be administered on a prophylactic basis in order to prevent the occurrence of arrhythmic episodes.

The compounds of Formula I slow the maximal rate of depolarization (phase 0), of the action potential of cardiac tissue. Thus, under the classification system of Vaughn Williams, these compounds exhibit a class I antiarrhythmic activity.

One method of demonstrating the antiarrhythmic activity of these compounds is the following test protocol. This protocol demonstrates what effect a compound has upon the action potential of isolated cardiac tissue, such as a Purkinje fiber from a dog heart or a papillary muscle from a guinea pig heart.

The heart of an anesthetized mongrel dog is surgically removed and the Purkinje fibers are dissected from either of the ventricles. Alternately, papillary muscles are removed from the right cardiac ventricle of a guinea pig. A Purkinje fiber or a papillary muscle is then placed in a tissue bath which is continuously

perfused with modified Tyrode's solution. [1]

The electrophysiology of the cardiac tissue is monitored by conventional glass microelectrodes. One microelectrode is inserted into a cell in the cardiac muscle fiber and a ground electrode is positioned in the tissue bath. A conventional oscilloscope is utilized to visualize the action potential waveforms of the cardiac cell.

The cardiac muscle fiber is electrically stimulated at a frequency of 1 Hz through a pair of platinum plates placed in the tissue bath. This stimulation is continued for approximately 1 hour in order to allow the electrophysiological characteristics of the fiber to stabilize.

After approximately 1 hour, the fiber should be exhibiting a stable action potential as demonstrated by the waveform displayed on the oscilloscope. At this point, representative control action potentials are recorded and analyzed by a computer.

After establishing a control action potential, the test compound is introduced into the Modified Tyrode's solution in a quantity such that the test compound is present within the tissue bath in a range of from $10^{-8}$ to $10^{-5}$ moles/liter. After the effect of the test compound has reached a steady state, the action potential is again recorded and analyzed in the manner described above. This analysis will demonstrate that the compounds of Formula I, such as, for example, endo-hexahydro-8-(3-indolylcarbonyloxy)-2,6-methano-2H-quinolizin-3(4H)-one are capable of delaying the rate of maximal depolarization of the action potential.

Another method of demonstrating the antiarrhythmic properties of the compounds of Formula I is by their ability to reduce the incidence of ventricular tachycardia and ventricular fibrillation that commonly occur when the coronary artery of a rat is occluded.

Male rats are utilized for the test protocol. On the day of the experiment, they should be anesthetized and artificially ventilated. An electrocardiogram (lead II) should be recorded and monitored on an ocilliscope.

The chest should be opened using a left thoracotomy followed by sectioning at ribs 4 and 5, 2 mm to the left of the sternum. After incising the pericardium, a 3/0 silk suture should be placed around the left main coronary artery, so that it can be occluded when desired. The heart should be repositioned in the thoracic cavity and the animal should be allowed a sufficient period of time to stabilize prior to further testing.

After the rat has stabilized, it should be administered the test compound intraperitoneally at a dosage range of from 5 mg/kg-25 mg/kg. Fifteen minutes later the coronary artery of a rat should be occluded by tightening the suture. The electrocardiogram should then be monitored for 15 minutes to detect the presence of ventricular tachycardia or ventricular fibrillations.

The protocol described above should be performed on a second rat so that it can serve as a control. Rather than being administered the test compound, it will be administered saline. The incidence of ventricular tachycardia and ventricular fibrillation will be significantly higher in the control group.

The compounds of Formula I are useful for treating a variety of arrhythmic conditions of the heart. Representative examples of arrhythmic conditions which are amendable to treatment with the compounds of the present invention include atrial tachycardia, atrial flutter, atrial fibrillation, premature ventricular contractions, and life threatening ventricular arrhythmias such as ventricular tachycardia, or ventricular fibrillation.

The compounds and medicaments of the present invention can be administered by a variety of routes. They are effective if administered either orally or parenterally (i.e., intravenously, intramuscularly, or subcutaneously).

The quantity of compound needed to either terminate an arrhythmic episode or prevent the occurrence of an arrhythmic episode (i.e., an antiarrhythmic quantity) will vary depending upon the route of administration, the patient, the severity of the patient's condition, the presence of other underlying disease states, and the particular compound utilized. However as a general guideline, if the compound is being administered orally, then it is preferably administered within a dosage range of from 1 mg to 10 mg/kg of patient body weight/day. Likewise, if the compound is being administered parenterally then it is preferably administered within a dosage range of from 1 mg to 7 mg/kg of patient body weight/day.

Repetitive daily administration of the compounds may be desired and will vary with the conditions outlined above for the quantity of compound utilized.

The patient's response to the compound can be monitored via an EKG or any other technique conventionally used in the art.

[1] The modified Tyrone's solution has the following composition (in mmol): NaCl 127.0, KCl 5.4, $NaH_2PO_4$ 0.5, $MgCl_2$ 1.0, $NaHCO_3$ 23.8, $CaCl_2$ 1.8 and glucose 11.1. A gas mixture comprised of 95% $O_2$ and 5% $CO_2$ is bubbled through the solution while it is maintained within a pH range of from 7.3-7.4.

As used in this application, the term patient refers to a warm-blooded animal. Thus, the compounds are effective for treating cardiac arrhythmias in mammals, such as humans, primates, sheep, horses, cattle, pigs, dogs, cats, rats, and mice, etc.

As used in this application, the term arrhythmia refers to any variation from the normal rhythm of the heart beat. Also as used in this application, the term antiarrhythmic refers to a compound capable of either preventing or alleviating an arrhythmia.

For oral administration, the compounds can be formulated into solid or liquid medicaments such as capsules, pills, tablets, lozenges, melts, powders, suspensions, or emulsions. Solid unit dosage forms can be capsules of the ordinary gelatin type containing, for example, surfactants, lubricants and inert fillers such as lactose, sucrose, and cornstarch or they can be sustained release medicaments. In another embodiment, the compounds of Formula I can be tableted with conventional tablet bases such as lactose, sucrose, and cornstarch in combination with binders, such as acacia, cornstarch, or gelatin, disintegrating agents such as potato starch or algenic acid, and a lubricant such as stearic acid or magnesium stearate. Liquid medicaments are prepared by dissolving the active ingredient in an aqueous or non-aqueous pharmaceutically acceptable solvent which may also contain suspending agents, sweetening agents, flavoring agents, and preservative agents as are known in the art.

For parenteral administration, the compounds may be dissolved in a physiologically acceptable pharmaceutical carrier and administered as either a solution or a suspension. Illustrative of suitable pharmaceutical carriers are water, saline, dextrose solutions, fructose solutions, ethanol, or oils of animal, vegetative, or synthetic origin. The pharmaceutical carrier may also contain preservatives, buffers, etc. as are known in the art.

The following examples are presented in order to further illustrate the present invention. However, they should not be construed as limiting the scope of the invention in any manner.

## EXAMPLE 1

To a stirred solution of 160 g of diethyl malonate in 1.5 l of dry dimethylformamide at 0 °C under nitrogen was slowly added 30 g of lithium hydride. After the evolution of hydrogen ceased (2 hours) 143 g of cis-1,4-dichloro-2-butene was slowly added and the mixture allowed to come to room temperature. After 72 hours, the mixture was diluted with a mixture of ether and hexane (1:4) and poured into water. The organic layer was washed with water and brine before drying over magnesium sulfate. Distillation gave diethyl 3-cyclopentene-1,1-dicarboxylate, bp 70-80 °C/0.1 mm, containing a small amount (~10%) of diethyl 2-vinylcyclopropane-1,1-dicarboxylate.

The impure cyclopentene diester (148.5 g) obtained above was added to a solution of 118 g of potassium hydroxide in 1333 ml of 80% ethanol and the stirred solution warmed at 60-70 °C overnight. The ethanol was evaporated and the residue treated with an ice cold solution of concentrated sulphuric acid (107 ml) in water (274 ml). Extraction of the acid mixture with ether (3 x 400 ml) followed by evaporation of the dried ether extracts gave a residue of the diacid which was decarboxylated to the monoacid by heating in an oil bath at 170-180 °C for 1 hour. The residual oil was distilled to give crude 3-cyclopentene1-carboxylic acid, bp 68-73 °C (1 mm) containing some γ-vinyl-γ-butyrolactone. A solution of 98 g of potassium carbonate in 300 ml of water was added and the mixture extracted with ether to remove the γ-vinyl-γ-butyrolactone. Acidification of the aqueous solution and extraction with ether afforded pure 3-cyclopentene-1-carboxylic acid.

## EXAMPLE 2

A mixture of 52 g of 3-cyclopentene-1-carboxylic acid and excess thionyl chloride was stirred at room temperature for 1 hour. The excess thionyl chloride evaporated and the residue distilled to give 3-cyclopentene-1-carbonyl chloride, bp 52-58 °C.

The acid chloride obtained above was slowly added to an ice cooled stirred solution of 32 g of pyridine in 150 ml of ethanol. The mixture was stirred for a further hour, the ethanol evaporated and the residue treated with water and ether. The ether layer was separated, washed several times with water and dried. Evaporation of the ether left a residue of ethyl 3-cyclopentene-1-carboxylate, bp 62.5-66 °C/14 mm.

EXAMPLE 3

A solution containing 84.6 g of N-methylmorpholine N-oxide, 1 g of osmium tetroxide, 230 ml of water and 115 ml of acetone was allowed to stir for 30 minutes at room temperature. To this stirred mixture was added very slowly over at least 8 hours, a solution of 80 g of ethyl 3-cyclopentene-1-carboxylate in 115 ml of acetone. The stirred mixture was heated at 50°C for 2 hours to complete the reaction (verified by TLC examination using ethyl acetate/hexane 70/30). Sodium bisulfite (~10 g) was added, the stirring continued for a further 15 minutes, and the mixture filtered through Celite. The pH of the filtrate was adjusted to 7 by the addition of 12 N sulfuric acid (37 ml), the acetone evaporated, the pH of the residual solution adjusted to 2 with 12 N sulfuric acid (13 ml) and the solution extracted with ethyl acetate (4 x 250 ml). Evaporation of the dried ethyl acetate solution gave 4-ethoxycarbonyl-1,2-cyclopentanediol.

EXAMPLE 4

A solution of 85.4 g of sodium periodate in 500 ml of water was slowly added to a stirred solution of 69 g of 4-ethoxycarbonyl-1,2-cyclopentanediol in 690 ml of tetrahydrofuran. The reaction was exothermic and cooling was necessary. After two hours a precipitate of sodium iodate was filtered off and the solution concentrated at room temperature to remove most of the tetrahydrofuran. The resulting aqueous solution contained the desired β-ethoxycarbonylglutaraldehyde and was used directly in the next reaction.

To a stirred suspension of 400 g of potassium hydrogenphthalate in 800 ml of water was added, in sequence, a solution of 80 g of acetonedicarboxylic acid in 1200 ml of water, a solution of 80 g of glycine ethyl ester hydrochloride in 400 ml of water, and finally the solution of β- ethoxycarbonylglutaraldehyde obtained above. The mixture was stirred for 20 hours at room temperature during which time carbon dioxide evolved. The mixture was basified by the addition of an excess of aqueous potassium carbonate and extracted with ethyl acetate several times. Evaporation of the dried ethyl acetate extracts gave a syrup consisting mainly of 7-ethoxycarbonyl-9-(ethoxycarbonylmethyl)-9-azabicyclo-[3.3.1]nonan-3-one.

EXAMPLE 5

Sodium borohydride (17 g) was added in small portions to a stirred solution of 87.6 g of 7-ethoxycarbonyl-9-(ethoxycarbonylmethyl)-9-azabicyclo[3.3.1]nonan-3-one in 750 ml of ethanol. The mixture was stirred overnight at room temperature, the ethanol evaporated and the residue treated with 200 ml of water. Hydrochloric acid (2 M) was added until the mixture was acid and this acid solution was immediately basified by the addition of saturated potassium carbonate solution. Extraction with ethyl acetate and evaporation of the dried extract gave a syrup which consisted mainly of 7-ethoxycarbonyl-9-(ethoxycarbonylmethyl)-9-azabicyclo[3.3.1]nonan-3-ol. The syrup can be purified by column chromatography using silica and elution with hexane-ethyl acetate (30:70).

EXAMPLE 6

A solution of 26.1 g of the crude 7-ethoxycarbonyl-9-(ethoxycarbonylmethyl)-9-azabicyclo[3.3.1]nonan-3-ol in 250 ml of methylene chloride was treated with one equivalent of methanesulfonic acid (8.42 g). The methylene chloride solution was concentrated to about 35 ml, 9.5 ml of dihydropyran was added together with one drop of methansulfonic acid, and the mixture stirred for 3 hours at room temperature. The mixture was then poured into saturated potassium carbonate solution and the product separated by extraction with ethyl acetate.

Evaporation of the dried ethyl acetate extracts gave a syrup consisting mainly of the tetrahydropyranyl ether of 7-ethoxy-carbonyl-9-(ethoxycarbonylmethyl)-9-azabicyclo[3.3.1]nonan-3-ol. It can be purified by column chromatography using silica and elution with hexane-ethyl acetate (20:80), Rf 0.7.

## EXAMPLE 7

A solution of 34 g of the tetrahydropyranyl ether of 7-ethoxycarbonyl-9-(ethoxycarbonylmethyl)-9-azabicyclo[3.3.1]nonan-3-ol in 800 ml of anhydrous toluene was treated with 19 g of potassium tert-butoxide and the stirred mixture heated at 100 ° C for 2 hours. Anhydrous formic acid (7.85 g) was added to the cooled mixture, the potassium formate was filtered off, and the toluene solution evaporated to give a syrup. The syrup was treated with 300 ml of 5 N hydrochloric acid and the stirred solution refluxed overnight. The cooled mixture was clarified by an extraction with methylene chloride and the aqueous acid solution evaporated to dryness. The residue was dissolved in a little water and the solution treated with a large excess of saturated potassium carbonate solution. Extraction of the resulting mixture with ethyl acetate and evaporation of the dried ethyl acetate solution gave endohexahydro-8-hydroxy-2,6-methano-2H-quinolizin-3-(4H)-one as an oil which crystallized on standing. The base was converted to its camphorsulfonate salt, m.p. 178 ° C, using one equivalent of camphorsulfonic acid in ethanol.

## EXAMPLE 8

A mixture of 1.8 g of endo-hexahydro-8-hydroxy-2,6-methano-2H-quinolizin-3(4H)-one, hydrofluoroboric acid (0.88 g; 60% aqueous solution) and 20 ml of ethanol was evaporated, the residue was treated with 50 ml of anhydrous toluene, and the mixture again evaporated. A stirred suspension of the anhydrous residue in 50 ml of anhydrous nitroethane at -78 ° C was treated with 1.94 g of anhydrous silver tetrafluoroborate. A solution of 1.8 g of 3-indolylcarbonyl chloride in 20 ml of anhydrous nitroethane was added slowly. The temperature of the stirred reaction was kept at -78 ° C for 1.5 hours and then allowed to return to room temperature overnight. Triethylamine (1 g) was added, the solution filtered and the nitroethane evaporated. A solution of the residue in 20 ml of water was treated with an excess of a saturated aqueous solution of potassium carbonate and the liberated oil separated by extraction with ethyl acetate. The ethyl acetate solution was washed several times with water before being dried over magnesium sulfate and evaporated. The residue obtained was endohexahydro-8-(3-indolylcarbonyloxy)-2,6-methano-2H-quinolizin-3(4H)-one and this was treated with an aqueous solution of methane sulfonic acid (1 equivalent) to give crystals of the methanesulfonate salt melting at about 278 ° C.

## EXAMPLE 9

When the procedure of Example VIII is repeated using endohexahydro-8-hydroxy-2,6-methano-2H-quinolizin-3(4H)-one and the appropriate acid chloride, the corresponding esters listed below are obtained. As necessary, the acid chlorides were obtained from the appropriate carboxylic acids by standard procedures, for example, using thionyl chloride. To convert the ester to a corresponding acid salt, it was reacted with the appropriate acid with alternative solvents being used as desired.

endo-8-(3-Benzofurancarbonyloxy)hexahydro-2,6-methano-2H-quinolozin-3(4H)-one
endo-8-(3-Benzo[b]thiophenecarbonyloxy)hexahydro-2,6-methano-2H-quinolizin-3(4H)-one
endo-8-(1-Benzyl-1H-indol-3-ylcarbonyloxy)hexahydro2,6-methano-2H-quinolizin-3(4H)-one
endo-Hexahydro-8-(1-methyl-1H-indol-3ylcarbonyloxy)2,6-methano-2H-quinolizin-3(4H)-one
endo-8-(4-Bromo-2-furylcarbonyloxy)hexahydro-2,6-methano-2H-quinolizin-3(4H)-one
endo-Hexahydro-8-(5-phenyl-2-furylcarbonyloxy)-2,6-methano-2H-quinolizin-3(4H)-one
endo-8-(3-Chloro-2-thienylcarbonyloxy)hexahydro-2,6-methano-2H-quinolizin-3(4H)-one
endo-Hexahydro-8-(5-methyl-2-thienylcarbonyloxy)-2,6-methano-2H-quinolizin-3(4H)-one
endo-Hexahydro-8-(1-methyl-1H-pyrrol-2-ylcarbonyloxy)2,6-methano-2H-quinolizin-3(4H)-one

## EXAMPLE X

Oxalyl chloride (0.76 ml) was slowly added to a stirred solution of 1 g of 5-methylindole in 20 ml of anhydrous ether at 0 ° c. The precipitate which formed was filtered off and dried at 80 ° C to give 5-methyl-3-indolylglyoxylyl chloride.

A stirred solution of 205 mg of anhydrous silver tetrafluoroborate in 10 ml of anhydrous nitroethane was treated with a solution of 282.5 mg of endo-hexahydro-8-hydroxy-2,6-methano-2H-quinolizin-3(4H)-one tetrafluoroborate (obtained by treating the free amine with an equivalent of hydrofluoroboric acid) in 10 ml of anhydrous nitroethane at room temperature. A solution of 233 mg of 5-methyl-3-indolylglyoxylyl chloride in 10 ml of anhydrous nitroethane was slowly added and the mixture stirred at room temperature overnight. Triethylamine (101 mg) was added, the solution filtered and the nitroethane evaporated. A solution of the residue in 15 ml of water was treated with a saturated aqueous solution of potassium carbonate and the liberated oil separated by extraction with ethyl acetate. The ethyl acetate solution was washed several times with water before being dried over magnesium sulfate and evaporated. The residue was treated with methylene chloride and ethereal hydrogen chloride, and the solid filtered off and recrystallized from 2-propanol to give endo-hexahydro-8-(5-methyl-3-indolylcarbonyloxy)-2,6-methano-2H-quinolizin-3(4H)-one hydrochloride.

When the above procedure was repeated using the appropriate substituted indole in place of the 5-methylindole, the following compounds were obtained:

endo-Hexahydro-8-(5-chloro-3-indolylcarbonyloxy)-2,6-methano-2H-quinolizin-3(4H)-one hydrochloride melting at about 317-320° C (with decomposition) after recrystallization from ethanol.

endo-Hexahydro-8-(5-cyano-3-indolylcarbonyloxy)-2,6-methano-2H-quinolizin-3(4H)-one hydrochloride melting at about 304-305° C (with decomposition) after recrystallization from ethanol.

endo-Hexahydro-8-(5-methoxy-3-indolycarbonyloxy)-2,6-methano-2H-quinolizin-3(4H)-one hydrochloride melting at about 303° C (with decomposition) after recrystallization from isopropanol.

exo-8-(3-indolycarbonyloxy)hexahydro-2,6-methane-2H-quinolizin-3(4H)-one melting at about 259-260° C after recrystallization from ethanol.

Also obtained in the same way are endo-hexahydro-8-(5-carbamoyl-3-indolylcarbonyloxy-2,6-methano-2H-quinolizin-3(4H)-one and endo-hexahydro-8-(5-hydroxy-3-indolycarbonyloxy)-2,6-methano-2H-quinolizin-3(4H)-one. In the later case, the starting material is 5-benzyloxyindole and the initial product is debenzylated by reduction using standard procedures.

## EXAMPLE 11

A solution of endo-8-(3-indolylcarbonyloxy)hexahydro-2,6-methano-2H-quinolizin-3(4H)-one (1.42 g) in ethanol (5 ml) was treated with fluoboric acid (0.64 g, 60% aqueous solution) and the mixture evaporated to give endo-8-(3-indolylcarbonyloxy)-hexahydro-2,6-methano-2H-quinolizin-3(4H)-one tetrafluoroborate (1.8 g).

A stirred suspension of the above salt (1.8 g) in anhydrous nitroethane (30 ml) was treated with propane-1,3-dithiol (3 ml) and boron trifluoride etherate (3 drops) and the mixture stirred overnight at room temperature. The nitroethane was removed by evaporation and the residue triturated with ether. The solid product was filtered off, washed several times with ether, treated with water (25 ml), saturated aqueous potassium carbonate (3 ml) and ether (50 ml). The ether solution was separated off, dried ($MgSO_4$) and evaporated to give the propane dithioketal derivative, m.p. 226-229° C (1.6 g).

Hydrazine hydrate (3 ml) was added dropwise during one hour to a stirred refluxing solution of the above dithioketal (0.5 g) in isopropanol (20 ml) in the presence of Raney nickel (6 g, previously washed three times with isopropanol). The reflux was maintained for a further 30 minutes, the hot solution filtered through a triple superphosphate, the nickel washed several times with hot isopropanol and the combined filtrates evaporated to give endo-8-(3-indolylcarbonyloxy)-2,6-methanooctahydro-2H-quinolizine as the free base (50 mg). Addition of methylene chloride and ethereal hydrogen chloride gave the hydrochloride (30 mg), m.p. 311-313° C (from ethanol).

## EXAMPLE 12

The procedure of Example 11 was repeated using endo hexahydro-8-hydroxy-2,6-methano-2H-quinolizin-3(4H)-one in place of the ester. The dithioketal obtained was reduced as described in the final paragraph except that the hydrazine hydrate was left out. This gave exo-octahydro-2,6-methano-2H-quinolizin-8-ol which was then reacted with 3-indolylcarbonyl chloride to give exo-8-(3-indoylcarbonyloxy)-octahydro 2,6-methano-2H-quinolizine which was converted to the hydrochloride, m.p. 255-256° C by

standard procedures.

## EXAMPLE 13

A stirred mixture of 1-methyl-3-indazolylcarboxylic acid (0.31 g), thionyl chloride (2 ml) and chloroform (10 ml) was refluxed for 2 hours and the solvent was evaporated to give a residue of 1-methyl-3-indazolylcarbonyl chloride.

A stirred solution of 395 mg of anhydrous silver tetrafluoroborate in anhydrous nitroethane (10 ml) was treated with a solution of endo-hexahydro-8-hydroxy-2,6-methano-2H-quinolizin-3(4H)-one tetrafluoroborate (475 mg) in anhydrous nitroethane (10 ml) at -78° C. A solution of 1-methyl-3-indazolylcarbonyl chloride (340 mg) in anhydrous nitroethane (5 ml) was slowly added during one hour and the reaction mixture was then allowed to warm to room temperature overnight. The mixture was poured into a saturated aqueous solution of potassium carbonate (30 ml). The mixture obtained was filtered and the separated solid was washed with ethyl acetate. The filtrate was then extracted twice with ethyl acetate (2 x 20 ml) and the solvent was evaporated from the combined ethyl acetate fractions. A solution of the residue in ethyl acetate (20 ml) was washed with water (3 x 15 ml) and dried over magnesium sulfate, and the solvent was evaporated to give a residual material. This material was purified by silica preparative plate chromatography using a mixture of ethanol/ethyl acetate (30:70) as eluant. The desired product compound, endo-hexahydro-8-(1-methyl-3-indazolylcarbonyloxy)-2,6-methano-2H-quinolizin- 3(4H)-one, formed a band with an Rf 0.35 and was isolated by extraction with ethanol/ethyl acetate (50:50).

## EXAMPLE 14

A stirred mixture of 690 mg of endo-hexahydro-8-(3-indolylcarbonyloxy)-2,6-methano-2H-quinolizin-3-(4H)-one, 400 mg of methyl iodide, and 100 ml of acetonitrile was refluxed for 2 hours and then allowed to stand overnight at room temperature. The crystalline solid which formed was separated by filtration and dried to give endo-hexahydro-8-(3-indolylcarbonyloxy)-2,6-methano-5-methyl-3(4H)-oxo-2H-quinolizinium iodide melting at about 310-312° C with decomposition.

## EXAMPLE 15

A mixture of 1.84 g of 4-quinolinecarboxylic acid, 25 ml of methylene chloride and trifluoroacetic anhydride was stirred at room temperature for 5 minutes and then cooled to 0° C. A mixture of 1.92 g of endo-hexahydro-8-hydroxy-2,6-methano-2H-quinolizin-3(4)-one, 1.2 g of trifluoroacetic acid, 25 ml of methylene chloride and 20 ml of tetrahydrofuran was slowly added and the mixture stirred at room temperature for 20 hours. The solid present was removed by filtration and the filtrate was basified by the addition of aqueous potassium carbonate. The resulting basic solution was extracted with ethyl acetate and the ethyl acetate extract was dried and filtered. The solvent was then evaporated to give residual material which was treated with ether and ethereal hydrogen chloride to give endo-hexahydro8-(4-quinolinylcarbonyloxy)-2,6-methano-2H-quinolizin-3(4H)-one hydrochloride melting at about 302° C (dec) after recrystallization from ethanol.

## EXAMPLE 16

A solution of endo-8-(3-indolylcarbonyloxy)hexahydro-2,6-methano-2H-quinolizin-3(4H)-one (50 mg) in 1 ml of ethanol was added dropwise to 20 mg of sodium borohydride in 1 ml of ethanol at room temperature. The reaction was then quenched by the addition of 2 ml of saturated aqueous ammonium chloride solution. The aqueous mixture was extracted three times with ethyl acetate and the solvent was evaporated from the combined organic extracts under nitrogen. This gave a solid residue which was endo-8-(3-indolylcarbonyloxy)-2,6-methanooctahydro-2H-quinolizin-3-ol.

## EXAMPLE 17

Oxalyl chloride (10 ml) was slowly added to a stirred solution of 11.7 g of indole in 50 ml of anhydrous ether at 0° C. The temperature was allowed to reach room temperature and the mixture stirred for a further 2 hours. The orange precipitate was filtered off, washed with anhydrous ether and dried at 50° C to give 3-indolylglyoxylyl chloride.

A suspension of 6.42 g of silver tetrafluoroborate in 300 ml of anhydrous toluene was evaporated to dryness to give a residue of the anhydrous salt. A solution of this anhydrous salt in anhydrous nitroethane (50 ml) was slowly added to a stirred solution of 7.74 g of trans-hexahydro-8-hydroxy-2,6-methano-2H-quinolizin-3(4H)-one tetrafluoroborate and 6 g of 3-indolylglyoxylyl chloride in 100 ml of anhydrous nitroethane cooled to -10° C under nitrogen. The mixture was stirred overnight at room temperature, poured into a saturated aqueous solution of potassium carbonate (30 ml) and the resulting mixture was extracted with 200 ml of ethyl acetate. The separated organic phase was dried over magnesium sulfate, the solvent evaporated and the residue redissolved in 200 ml of ethyl acetate. After washing three times with water to remove unchanged starting alcohol, the ethyl acetate solution was dried and evaporated to give a residue (7.4 g) of crude trans-hexahydro-8-(3-indolylcarbonyloxy)-2,6-methano-2H-quinolizin-3(4H)-one base. The residue was treated with a solution of 2.2 g of methanesulphonic acid in 50 ml of ethanol at 60° C. The solid material remaining undissolved was filtered off and the reddish-brown solution was treated with charcoal. On cooling, the filtered solution afforded crystals of trans-hexahydro-8-(3-indolylcarbonyloxy)-2,6-methano-2H-quinolizin-3(4H)-one methanesulphonate monohydrate (66%).

## Claims

1. The use in the manufacture of a medicament for the treatment of cardiac arrhythmias, of compounds of the following general formula:

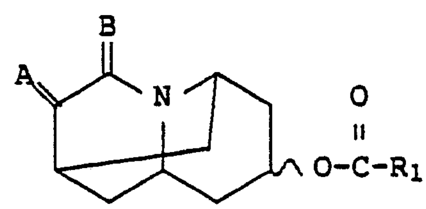

Formula I

wherein A is $H_2$, O, (H)(OH), $(OH)_2$ or N-OH; B is $H_2$, $(H)(CH_3)$, $(H)(CH_2NR_3R_4)$ or $CH_2$ wherein $R_3$ and $R_4$ are $C_{2-4}$ alkyl or are combined to give tetramethylene, pentamethylene or $-CH_2CH_2-O-CH_2CH_2-$; $R_1$ is

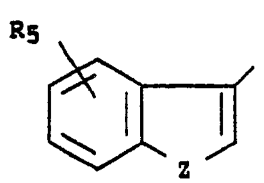 , 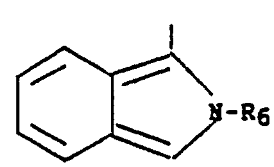 , 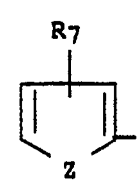

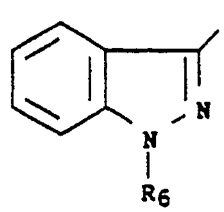 or 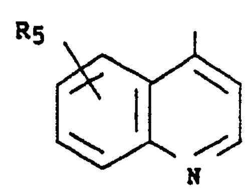

wherein Z is $NR_6$, O or S; $R_6$ is hydrogen, $C_{1-4}$alkyl or phenyl ($C_{1-12}$alkyl); $R_5$ is hydrogen, halogen, $C_{1-4}$

alkyl, $C_{1-4}$ alkoxy, hydroxy, cyano or $-CONH_2$; $R_7$ is hydrogen, halogen, $C_{1-4}$ alkyl or phenyl; the wavy line indicates that the configuration of the oxygen substituent on the ring can be endo or exo; and the pharmaceutically acceptable acid addition and quaternary ammonium salts of the aforesaid compounds.

2. Use according to claim 1 of a compound of formula I wherein the $R^1COO$ group is attached to the polycyclic ring in the endo configuration.

3. Use according to claim 1 of a compound of formula I wherein the $R^1COO$ group is attached to the polycyclic ring in the endo configuration and A represents O or $(OH)_2$.

4. Use according to claim 1 of a compound of formula I wherein the $R^1COO$ group is attached to the polycyclic ring in the endo configuration, A represents O or $(OH)_2$ and B represents $H_2$.

5. Use according to claim 1 of a compound of claims 1, 2, 3 or 4 wherein $R_1$ is

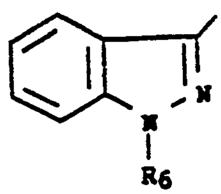

or

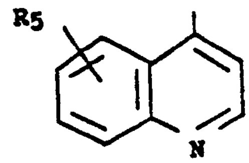

6. Use according to claim 1 of a compound of formula I which is endo-hexahydro-8-(3-indolylcarbonyloxy)-2,6--methano-2H-quinolizin-3(4H)-one.

7. Use according to claim 1 of a compound of formula I which is endo-8-(3-indolylcarbonyloxy)-octahydro-2,6--methano-2H-quinolizine.

8. Use according to claim 1 of a compound of formula I which is endo-8-(5-cyano-3-indolylcarbonyloxy)-hexahydro-2,6-methano-2H-quinolizin-3(4H)-one.

9. Use according to claim 1 of a compound of formula I which is endo-8-(3-indolylcarbonyloxy)-hexahydro--4-(diethylaminomethyl)-2,6-methano-2H-quinolizin--3(4H)-one.

10. Use according to claim 1 of a compound of formula I which is endo-8-(2-methyl-1-isoindolylcarbonyloxy)hexahydro-2,6-methano-2H-quinolizin-3(4H)-one.

European Patent Office

# EUROPEAN SEARCH REPORT

Application Number

EP 89 10 0964

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A<br><br>Y | EP-A-0 220 011 (BEECHAM GROUP PLC)<br>* Summary *<br><br>--- | 1-4,6-10<br>1-5 | A 61 K 31/435<br>A 61 K 31/47 |
| A,P<br>D<br>Y,D | EP-A-0 266 730 (MERRELL DOW)<br>* Claims *<br><br>----- | 1-4,6-10<br>1-5 | |
| | | | **TECHNICAL FIELDS SEARCHED (Int. Cl.4)** |
| | | | A 61 K 31/00 |

The present search report has been drawn up for all claims

| Place of search | Date of completion of the search | Examiner |
|---|---|---|
| THE HAGUE | 01-03-1989 | THEUNS H.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document